# EUROPEAN PATENT APPLICATION

(11) **EP 4 818 151 A1**
(43) Date of publication of application: **07.10.2026**
(21) Application number: 26169373.3
(22) Date of filing: 31.03.2026
(51) Int. Cl.: C07C 227/14, A23L 33/165, A61K 9/20, A61K 33/08, C01F 5/14, C07C 227/42, C07C 229/08, C07C 229/76

(54) **MAGNESIUM GLYCINATE**

(30) Priority: 31.03.2025 JP 2025058711
(71) Applicant: SETOLAS Holdings, Inc., Takamatsu-shi, Kagawa 760-0026 (JP)
(72) Inventor: NOMURA, Satoshi, Takamatsu-shi, Kagawa, 760-0026 (JP); KUNIMURA, Naoto, Takamatsu-shi, Kagawa, 760-0026 (JP); WAKAMATSU, Junichiro, Takamatsu-shi, Kagawa, 760-0026 (JP)
(74) Representative: Vossius & Partner Patentanwälte Rechtsanwälte mbB

(57) **Abstract**

An object of the present disclosure is to provide magnesium glycinate having good compression moldability. Magnesium glycinate having a crystallinity represented by S2/(S1 + S2) of 0 or more and 0.9 or less, wherein in an X-ray powder diffraction spectrum, S1 is an area of an amorphous peak having a maximum value in a range of 20° ≤ 2θ ≤ 30° and a full width at half maximum of 2° or more, and S2 is an area obtained by subtracting a sum of areas of peaks attributed to magnesium hydroxide in a range of 5° ≤ 2θ ≤ 70° from a sum of areas of crystal peaks having a maximum value in a range of 5° ≤ 2θ ≤ 70° and a full width at half maximum of less than 1°.

## Description

### TECHNICAL FIELD

The present disclosure relates to magnesium glycinate and a method for producing the same.

### BACKGROUND ART

Magnesium glycinate is excellent in bioabsorbability and has decreased side effects such as a laxative effect, and thus can be used for food or beverage products, nutraceutical products, and pharmaceutical products and the like.

Patent Document 1 describes a magnesium glycinate hydrate having a structure represented by the following formula.

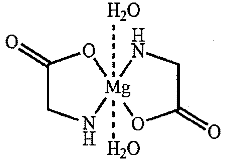

Patent Document 2 describes a method in which an organic acid ligand and a metal compound are added to a nonaqueous liquid to form a suspension, next the organic acid ligand and the metal compound are reacted, further the suspension is heated while being stirred for a predetermined time, and the suspension is filtered to obtain an insoluble metal organic chelate.

Patent Document 3 describes a method in which a solvent-free mixture of a metal compound and a solid organic acid comprising a chelate-forming acid such as α- and β-amino acids or a hydroxycarboxylic acid is exposed to a strong mechanical stress to induce a solid reaction that forms a metal chelate.

### Patent Documents

Patent Document 1: US 2014/0186408 A
Patent Document 2: JP 2005-145822 A
Patent Document 3: JP 2020-517602 A

### SUMMARY

### Problems to be Solved by the Invention

Conventional magnesium glycinates are usually present as crystals. As these crystals have insufficient compression moldability, there are drawbacks such as an increase in size per tablet and an increase in the number of tablets for obtaining a necessary effect when trying to produce tablets using these crystals. The present disclosure was made in view of such circumstances, and an object thereof is to provide magnesium glycinate having good compression moldability.

### Solutions to the Problems

A first embodiment of the present disclosure provides magnesium glycinate having a crystallinity represented by S2/(S1 + S2) of 0 or more and 0.9 or less, wherein in an X-ray powder diffraction spectrum, S1 is an area of an amorphous peak having a maximum value in a range of 20° ≤ 2θ ≤ 30° and a full width at half maximum of 2° or more, and S2 is an area obtained by subtracting a sum of areas of peaks attributed to magnesium hydroxide in a range of 5° ≤ 2θ ≤ 70° from a sum of areas of crystal peaks having a maximum value in a range of 5° ≤ 2θ ≤ 70° and a full width at half maximum of less than 1°.

In a second embodiment of the present disclosure, in the first embodiment, a weight reduction rate at 400°C based on a weight at 50°C in the thermogravimetric analysis of the magnesium glycinate may be 40% by mass or less.

In a third embodiment of the present disclosure, in any one of the first and second embodiments, the crystallinity of the magnesium glycinate may be 0 or more and 0.81 or less.

A fourth embodiment of the present disclosure provides a food or beverage product, or a pharmaceutical product comprising the magnesium glycinate according to any one of the first to third embodiments.

A fifth embodiment of the present disclosure provides a method for producing a food or beverage product, or a pharmaceutical product using the magnesium glycinate according to any one of the first to third embodiments.

A sixth embodiment of the present disclosure provides a magnesium glycinate tablet comprising the magnesium glycinate according to any one of the first to third embodiments.

A seventh embodiment of the present disclosure provides a magnesium glycinate tablet having a tensile strength of 100 N/cm² or more and 500 N/cm² or less when tableted at a tableting pressure of 5 kN per tablet.

In the seventh embodiment, the tensile strength of the magnesium glycinate tablet when tableted at a tableting pressure of 5 kN per tablet may be 100 N/cm² or more and 500 N/cm² or less.

An eighth embodiment of the present disclosure provides a method for producing magnesium glycinate comprising a step of obtaining a magnesium glycinate by reacting glycine with a magnesium raw material, wherein the magnesium raw material comprises one or more selected from magnesium hydroxide and magnesium oxide.

A ninth embodiment of the present disclosure provides a method for producing magnesium glycinate comprising a step of obtaining the magnesium glycinate by spray-drying a slurry comprising glycine and a magnesium raw material, wherein the magnesium raw material comprises one or more selected from magnesium hydroxide and magnesium oxide, wherein a solid content concentration of the slurry is 9.1% by mass or more and 41.7% by mass or less, and wherein a slurry temperature immediately before spraying is 20°C or higher and 90°C or lower.

In a tenth embodiment of the present disclosure, in either of the eighth or ninth embodiments, the magnesium raw material may comprise magnesium hydroxide.

In an eleventh embodiment of the present disclosure, in any one of the eighth to tenth embodiments, a total content of the iron and manganese in the magnesium raw material may be 0 ppm by mass or more and 270 ppm by mass or less, with respect to the total of the magnesium raw material.

In a twelfth embodiment of the present disclosure, in any one of the eighth to eleventh embodiments, the magnesium raw material may comprise magnesium hydroxide and magnesium oxide. The content of the magnesium hydroxide may be 20% by mass or more in the magnesium raw material.

### Effects of the Invention

The magnesium glycinate of the present disclosure exhibits good compression moldability.

### BRIEF DESCRIPTION OF DRAWINGS

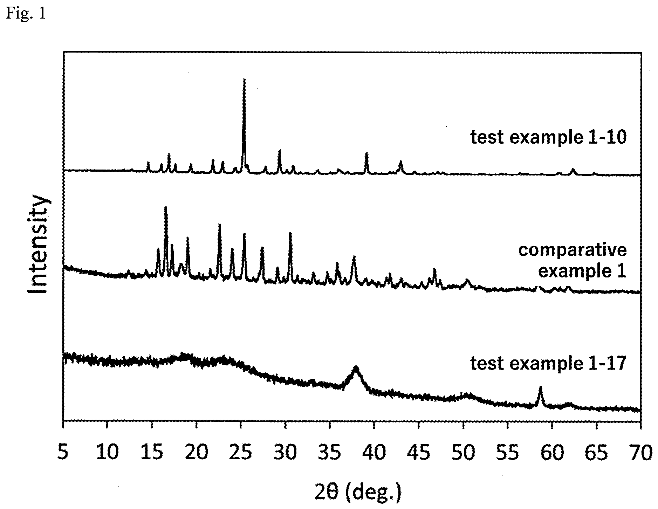

Fig. 1 illustrates the powder X-ray diffraction spectra of test example 1-10, test example 1-17, and comparative example 1;

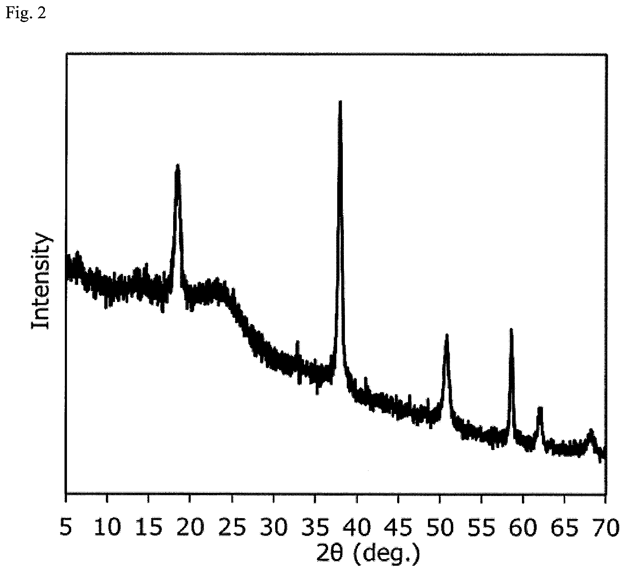

Fig. 2 illustrates the powder X-ray diffraction spectrum of magnesium hydroxide;

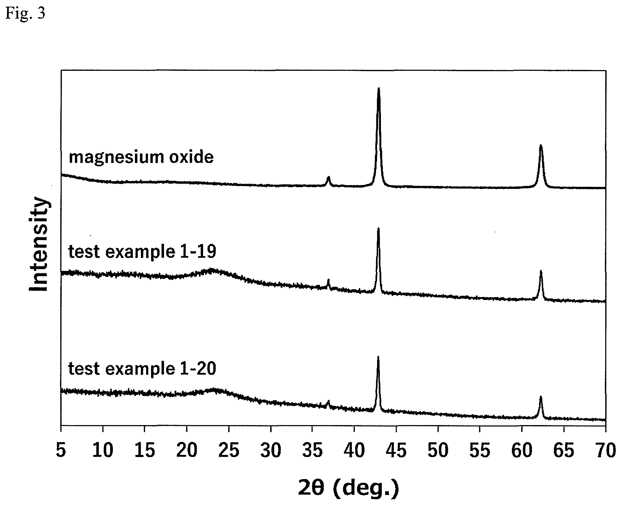

Fig. 3 illustrates the powder X-ray diffraction spectra of magnesium oxide, test example 1-19, and test example 1-20.

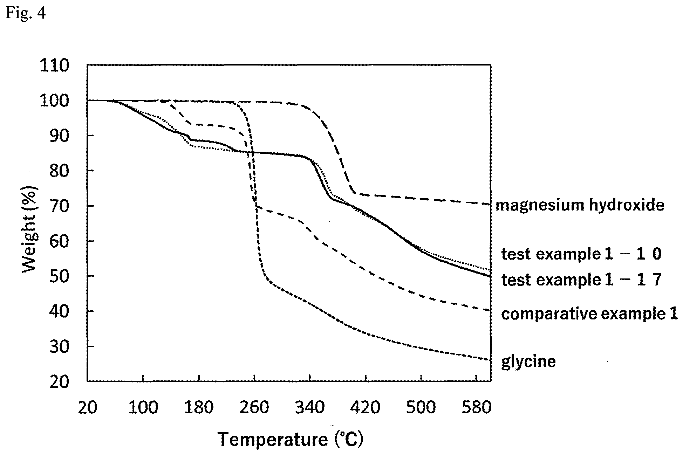

Fig. 4 illustrates the thermogravimetric analysis curves of magnesium hydroxide, glycine, test example 1-10, test example 1-17, and comparative example 1; and
Fig. 5 is a diagram illustrating an example of peak fitting in calculating a crystallinity.

### DETAILED DESCRIPTION

The present disclosure relates to magnesium glycinate. It should be noted that, in the present specification, various numerical ranges mean ranges comprising upper and lower limit values unless otherwise specified.

Magnesium glycinate as a compound is considered to have a structure in which two ligands having a structure where one hydrogen atom is eliminated from glycine are bonded to one magnesium atom. The magnesium glycinate may be present as an amorphous or a crystal.

In the present disclosure, when simply referred to as magnesium glycinate, the magnesium glycinate is not limited to magnesium glycinate as a compound, and comprises a material comprising a component other than magnesium glycinate as a compound. In such a material, the form of the magnesium glycinate is not particularly limited, and may comprise either the crystal or the amorphous.

### (First Embodiment: Magnesium Glycinate)

The magnesium glycinate of the present disclosure has a crystallinity represented by S2/(S1 + S2) (hereinafter, also simply referred to as a "crystallinity") of 0 or more and 0.9 or less, wherein in an X-ray powder diffraction (XRD) spectrum, S1 is an area of an amorphous peak having a maximum value in a range of 20° ≤ 2θ ≤ 30° and a full width at half maximum (hereinafter, also referred to as "FWHM") of 2° or more, and S2 is an area obtained by subtracting a sum of areas of peaks attributed to magnesium hydroxide in a range of 5° ≤ 2θ ≤ 70° from a sum of areas of crystal peaks having a maximum value in a range of 5° ≤ 2θ ≤ 70° and a full width at half maximum of less than 1°.

The magnesium glycinate of the present disclosure exhibits good compression moldability. Accordingly, the use of the magnesium glycinate of the present disclosure is expected to reduce the use of additives necessary for molding, and provide a tablet having a high magnesium glycinate concentration. The tensile strength of the tablet obtained from the magnesium glycinate of the present disclosure is good. Therefore, the magnesium glycinate is expected to provide a tablet by tableting, and the tablet may be distributed while being stably maintained. As a result, magnesium glycinate may be provided in a form of a tablet while it has been conventionally sold as a hard capsule, and the burden on a user in terms of the number and size of the tablets to be taken is expected to be reduced. The present disclosure should not be construed as being limited to a specific theory, but the reason the magnesium glycinate of the present disclosure exhibits the above effect is considered as follows.

The magnesium glycinate of the present disclosure is considered to have a low crystallinity and a high amorphous proportion. As a result, when subjected to a compressive stress, the magnesium glycinate is considered to be deformed more easily than the crystal, and exhibits good compression moldability.

The crystallinity of the magnesium glycinate is preferably 0 or more and 0.9 or less, more preferably 0 or more and 0.87 or less, and still more preferably 0 or more and 0.81 or less. When the crystallinity is in such a range, the compression moldability is better. In a preferred embodiment, the crystallinity of the magnesium glycinate is preferably 0 or more and 0.95 or less, more preferably 0.5 or more and 0.93 or less, and still more preferably 0.7 or more and 0.9 or less.

The amorphous peak is considered to be a peak attributed to amorphous magnesium glycinate. The range of 2θ where the maximum value of the amorphous peak is present is 20° or more and 30° or less, preferably 20.5° or more and 29° or less, and more preferably 21° or more and 28° or less in an X-ray powder diffraction (XRD) spectrum.

The full width at half maximum of the amorphous peak is 2° or more, preferably 2.2° or more, and more preferably 2.4° or more. The upper limit of the range is not limited, but is preferably 20° or less, more preferably 18° or less, and still more preferably 16° or less in an X-ray powder diffraction (XRD) spectrum.

The crystal peak is considered to be a peak derived from a crystal component. Examples of the crystal component include crystals of the magnesium glycinate and crystals of other components such as magnesium hydroxide. The range of 2θ where the maximum value of the crystal peak is present is 5° or more and 70° or less, preferably 10° or more and 50° or less, and more preferably 12° or more and 45° or less in an X-ray powder diffraction (XRD) spectrum.

The full width at half maximum of the crystal peak is more than 0° and less than 1°, more preferably more than 0° and 0.8° or less, and still more preferably more than 0° and 0.6° or less. The lower limit thereof is not limited, but is more than 0°, preferably 0.05° or more, and still more preferably 0.08° or more in an X-ray powder diffraction (XRD) spectrum.

In the present disclosure, the XRD spectrum of the magnesium glycinate may be measured by a powder X-ray diffraction method. The powder X-ray diffraction measurement can be performed at an acceleration voltage of 45 kV, and an angle step of 0.026° using a CuKα ray (λ = 1.5418 Å) as an X-ray source.

When the magnesium glycinate of the present disclosure comprises glycine crystals, the magnesium glycinate crystal preferably comprises an α-type glycine crystal. The α-type glycine crystal is a metastable crystal form, and chelation proceeds more readily compared to a stable γ-type crystal. Consequently, the crystallinity of the magnesium glycinate as a whole is considered to contribute to controlling the crystallinity towards being enhanced. The α-type crystals may be those exhibiting in an X-ray powder diffraction (XRD) spectrum one or more (preferably all) peaks in the 2θ range of, for example, preferably 18.8° or more and 19.2° or less, 23.7° or more and 24.1° or less, and 35.1° or more and 35.5° or less. The γ-type crystal may, for example, be a crystal exhibiting one or more (preferably all) peaks in an X-ray powder diffraction (XRD) spectrum at 20 values selected from the ranges of preferably 21.5° or more and 21.9° or less, 25.0° or more and 25.4° or less, and 35.7° or more and 35.9° or less.

The magnesium glycinate of the present disclosure may have a weight reduction rate at 400°C of preferably 0% by mass or more and 40% by mass or less, more preferably 10% by mass or more and 40% by mass or less, and still more preferably 20% by mass or more and 40% by mass or less, based on a weight at 50°C in thermogravimetric analysis. A weight reduction in a range of 50°C to 400°C is considered to be derived from free water and hydration water in the magnesium glycinate, the combustion of glycine present in the magnesium glycinate as an impurity, and the combustion of magnesium glycinate as a compound. In the present disclosure, the thermogravimetric analysis can be performed at a heating rate of 10°C/min in a range of 30°C to 600°C under a nitrogen atmosphere.

The content of magnesium glycinate as a compound in the magnesium glycinate of the present disclosure is preferably 85% by mass or more and 100% by mass or less, more preferably 87% by mass or more and 99% by mass or less, and still more preferably 90% by mass or more and 98% by mass or less, based on the total amount of the magnesium glycinate (magnesium glycinate as a material) of the present disclosure excluding moisture.

The content of magnesium glycinate as a compound in the magnesium glycinate may be calculated on the basis of the thermogravimetric analysis. Specifically, the content can be calculated as 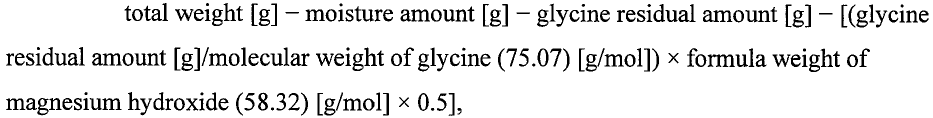 total weight [g] - moisture amount [g] - glycine residual amount [g] - [(glycine residual amount [g]/molecular weight of glycine (75.07) [g/mol]) × formula weight of magnesium hydroxide (58.32) [g/mol] × 0.5], and the content is expressed as a percentage of the value with respect to a value (total weight - moisture amount) obtained by subtracting the moisture amount from the total weight. Here, the amount of the hydration water can be measured as a difference between a weight at 180°C and a weight at 50°C when the thermogravimetric analysis is performed on the magnesium glycinate at a heating rate of 10°C/min, and the glycine residual amount can be similarly measured as a difference between a weight at 200°C and a weight at 280°C.

The magnesium glycinate of the present disclosure may comprise a component other than magnesium glycinate as a compound. Examples of such a component include magnesium hydroxide. The content of the magnesium hydroxide in the magnesium glycinate of the present disclosure is preferably 0% by mass or more and 10% by mass or less, more preferably 0% by mass or more and 5% by mass or less, and still more preferably 0% by mass or more and 3% by mass or less, based on the total amount of the magnesium glycinate (magnesium glycinate as a material) of the present disclosure.

The content of the magnesium hydroxide in the magnesium glycinate (magnesium glycinate as a material) of the present disclosure can be quantitatively analyzed by using a least squares method or a reference intensity ratio (RIR) method, or the like in X-ray powder diffraction measurement. Alternatively, theoretically, since the glycine and the magnesium hydroxide are chelated at a molar ratio of 2 : 1, a half amount ((glycine residual amount [g]/molecular weight of glycine (75.07) [g/mol]) × formula weight of magnesium hydroxide (58.32) [g/mol] ×0.5) as the molar ratio of the glycine residual amount is the residual amount of the magnesium hydroxide. The content can be calculated based on the residual amount of the magnesium hydroxide.

The magnesium glycinate of the present disclosure may be in the form of a powder, a particle, or a granule or the like. The average particle diameter of the magnesium glycinate of the present disclosure is preferably 1 µm or more and 350 µm or less, more preferably 10 µm or more and 300 µm or less, still more preferably 20 µm or more and 250 µm or less, and yet still more preferably 30 µm or more and 100 µm or less. In the present disclosure, the average particle diameter means a volume-based median diameter (D50), and can be measured by a laser diffraction/scattering method.

### (Second Embodiment: Method for Producing Magnesium Glycinate)

The magnesium glycinate of the present disclosure may be produced by a production method comprising the step of obtaining the magnesium glycinate by reacting glycine with a magnesium raw material.

The magnesium raw material comprises one or more selected from magnesium hydroxide and magnesium oxide.

According to the above production method, the magnesium glycinate having good compression deformability can be produced. The present disclosure should not be construed as being limited to a specific theory, but the reason why such an effect can be obtained by the production method of the present disclosure is considered as follows. That is, it is considered that when the glycine is reacted with the magnesium raw material, the rate of a chelation reaction is reduced, and as a result, crystal growth is inhibited, and not a crystal but an amorphous is easily generated. As a result, it is considered that the magnesium glycinate having good compression deformability is easily obtained.

Glycine is preferably 0.32 parts by mass or more and 7.7 parts by mass or less, more preferably 0.64 parts by mass or more and 6.4 parts by mass or less, and most preferably 1.3 parts by mass or more and 5.1 parts by mass or less, based on one part by mass of magnesium raw material. Having the glycine quantity within the above range relative to the magnesium raw material quantity enhances reaction efficiency and facilitates achieving a higher magnesium concentration in the resulting magnesium glycinate.

The glycine is preferably 0.32 parts by mass or more and 7.7 parts by mass or less, more preferably 0.64 parts by mass or more and 6.4 parts by mass or less, and still more preferably 1.3 parts by mass or more and 5.1 parts by mass or less, with respect to 1 part by mass of the magnesium hydroxide. When the amount of the glycine is within the above range with respect to the amount of the magnesium hydroxide, it is easier to increase the reaction efficiency and to further increase a magnesium concentration in the obtained magnesium glycinate.

The glycine is preferably 1.4 mol or more and 2.6 mol or less, more preferably 1.6 mol or more and 2.4 mol or less, and still more preferably 1.8 mol or more and 2.2 mol or less, with respect to 1 mol of magnesium comprised in the magnesium raw material.

Glycine may comprise glycine crystals. Examples of glycine crystals include α-type, β-type, and γ-type. It is preferable for the glycine crystals to comprise α-type glycine crystals. The α-type is a metastable crystal form; compared to the stable γ-type crystal, chelation proceeds more readily. Consequently, it is considered possible to control the resulting magnesium glycinate towards enhanced crystallinity.

The magnesium raw material is typically an inorganic compound comprising magnesium, and comprises one or more selected from magnesium hydroxide and magnesium oxide.

In one embodiment, the magnesium raw material preferably comprises magnesium hydroxide. The content of the magnesium hydroxide is preferably 27% by mass or more and 100% by mass or less, more preferably 60% by mass or more and 100% by mass or less, and still more preferably 80% by mass or more and 100% by mass or less, with respect to the magnesium raw material.

In another embodiment, the magnesium raw material comprises magnesium oxide. The content of the magnesium oxide is preferably 27% by mass or more and 100% by mass or less, more preferably 60% by mass or more and 100% by mass or less, and still more preferably 80% by mass or more and 100% by mass or less, with respect to the magnesium raw material.

In yet another embodiment, the magnesium raw material may further comprise magnesium hydroxide and magnesium oxide. When comprising magnesium hydroxide and magnesium oxide, the content of the magnesium hydroxide is preferably 20% by mass or more and 100% by mass or less, more preferably 27% by mass or more and 100% by mass or less, even more preferably 60% by mass or more and 100% by mass or less, and still more preferably 65% by mass or more and 100% by mass or less, with respect to the magnesium raw material.

The content of the magnesium oxide in the magnesium raw material is preferably 0 parts by mass or more and 270 parts by mass or less, more preferably 0 parts by mass or more and 150 parts by mass or less, and still more preferably 0 parts by mass or more and 100 parts by mass or less, with respect to 100 parts by mass of the magnesium hydroxide in the magnesium raw material.

The molar ratio (magnesium oxide : magnesium hydroxide) of the magnesium oxide and the magnesium hydroxide in the magnesium raw material is preferably 0 : 10 to 8 : 2, more preferably 0 : 10 to 7 : 4, and still more preferably 0 : 10 to 6 : 4. Reducing the proportion of magnesium oxide tends to lower the degree of crystallinity, whereas a higher proportion of magnesium oxide tends to increase the degree of crystallinity.

The magnesium raw material may preferably have low heavy metal content. The low content of the heavy metal content in the magnesium raw material suppresses the formation of magnesium glycinate crystal nuclei, thereby facilitating the production of magnesium glycinate with low crystallinity.

The examples of the heavy metal are not limited, but may include chromium, manganese, iron, cobalt, nickel, copper, zinc, molybdenum, ruthenium, rhodium, palladium, silver, cadmium and tin.

The iron content in the magnesium raw material is preferably 0 ppm by mass or more and 200 ppm by mass or less, more preferably 0 ppm by mass or more and 150 ppm by mass or less, and more preferably 0 ppm by mass or more and 100 ppm by mass or less with respect to the total of the magnesium raw material. The iron content within this range in the magnesium raw material is expected to result in low concentration of heavy metals within the magnesium raw material, thereby facilitating the suppression of crystallinity in the resulting magnesium glycinate.

The manganese content in the magnesium raw material is preferably 0 mass ppm or more and 70 mass ppm or less, more preferably 0 mass ppm or more and 50 mass ppm or less, and most preferably 0 mass ppm or more and 30 mass ppm or less with respect to the total of the magnesium raw material. The manganese content within this range in the magnesium raw material is expected to result in low concentrations of heavy metals within the magnesium raw material, thereby facilitating the suppression of crystallinity in the resulting magnesium glycinate.

The total content of iron and manganese in the magnesium raw material is preferably 0 ppm by mass or more and 270 ppm by mass or less, more preferably 0 ppm by mass or more and 200 ppm by mass or less, and more preferably 0 ppm by mass or more and 130 ppm by mass or less with respect to the total of the magnesium raw material. The total content of iron and manganese within this range in the magnesium raw material is expected to result in low concentration of heavy metals within the magnesium raw material, thereby facilitating the suppression of crystallinity in the resulting magnesium glycinate.

The iron content in the magnesium hydroxide is preferably 0 ppm by mass or more and 100 ppm by mass or less, more preferably 0 ppm by mass or more and 50 ppm by mass or less, and more preferably 0 ppm by mass or more and 25 ppm by mass or less with respect to the total of the magnesium hydroxide. The iron content within this range in the magnesium hydroxide is expected to result in low concentration of heavy metals within the magnesium hydroxide, thereby facilitating the suppression of crystallinity in the resulting magnesium glycinate.

The manganese content in the magnesium hydroxide is preferably 0 ppm by mass or more and 20 ppm by mass or less, more preferably 0 ppm by mass or more and 10 ppm by mass or less, and more preferably 0 ppm by mass or more and 5 ppm by mass or less with respect to the total of the magnesium hydroxide. The manganese content within this range in the magnesium hydroxide is expected to result in low concentration of heavy metals within the magnesium hydroxide, thereby facilitating the suppression of crystallinity in the resulting magnesium glycinate.

The total content of iron and manganese in the magnesium hydroxide is preferably 0 ppm by mass or more and 120 ppm by mass or less, more preferably 0 ppm by mass or more and 60 ppm by mass or less, and more preferably 0 ppm by mass or more and 30 ppm by mass or less with respect to the total of the magnesium hydroxide. The total content of iron and manganese within this range in the magnesium hydroxide is expected to result in low concentration of heavy metals within the magnesium hydroxide, thereby facilitating the suppression of crystallinity in the resulting magnesium glycinate.

The iron content in the magnesium oxide is preferably 0 ppm by mass or more and 200 ppm by mass or less, more preferably 0 ppm by mass or more and 100 ppm by mass or less, and more preferably 0 ppm by mass or more and 50 ppm by mass or less with respect to the total of the magnesium oxide. The iron content within this range in the magnesium oxide is expected to result in low concentration of heavy metals within the magnesium oxide, thereby facilitating the suppression of crystallinity in the resulting magnesium glycinate.

The manganese content in the magnesium oxide is preferably 0 ppm by mass or more and 40 ppm by mass or less, more preferably 0 ppm by mass or more and 20 ppm by mass or less, and more preferably 0 ppm by mass or more and 10 ppm by mass or less with respect to the total of the magnesium oxide. The manganese content within this range in the magnesium oxide is expected to result in low concentration of heavy metals within the magnesium oxide, thereby facilitating the suppression of crystallinity in the resulting magnesium glycinate.

The total content of iron and manganese in the magnesium oxide is preferably 0 ppm by mass or more and 240 ppm by mass or less, more preferably 0 ppm by mass or more and 120 ppm by mass or less, and more preferably 0 ppm by mass or more and 60 ppm by mass or less with respect to the total of the magnesium oxide. The total content of iron and manganese within this range in the magnesium oxide is expected to result in low concentration of heavy metals within the magnesium oxide, thereby facilitating the suppression of crystallinity in the resulting magnesium glycinate.

In this disclosure, a heavy metal content may be measured by high-frequency inductively coupled plasma (ICP) emission spectroscopy.

A reaction between the glycine and the magnesium raw material is preferably performed by spray-drying a slurry comprising the glycine and the magnesium raw material. It is considered that the crystallization of the glycine and the magnesium raw material after the chelation reaction can be suppressed by spray drying, and the amorphous is more easily generated.

The content of the magnesium raw material is preferably 20% by mass or more and 40% by mass or less, more preferably 20% by mass or more and 30% by mass or less, and still more preferably 25% by mass or more and 30% by mass or less, with respect to the solid content in the slurry.

The iron content in the slurry is preferably 0 ppm by mass or more and 20 ppm by mass or less, more preferably 0 ppm by mass or more and 15 ppm by mass or less, and most preferably 0 ppm by mass or more and 10 ppm by mass or less with respect to the total of the slurry. With the iron content in the slurry falling within this range, it is anticipated that the concentration of heavy metals within the slurry will also be low, thereby facilitating the suppression of crystallinity in the resulting magnesium glycinate.

The manganese content in the slurry is preferably 0 ppm by mass or more and 10 ppm by mass or less, more preferably 0 ppm by mass or more and 8 ppm by mass or less, and most preferably 0 ppm by mass or more and 6 ppm by mass or less with respect to the total of the slurry. With the manganese content in the slurry falling within this range, it is anticipated that the concentration of heavy metals within the slurry will also be low, thereby facilitating the suppression of crystallinity in the resulting magnesium glycinate.

The total content of iron and manganese in the slurry is preferably 0 ppm by mass or more and 30 ppm by mass or less, more preferably 0 ppm by mass or more and 23 ppm by mass or less, and most preferably 0 ppm by mass or more and 16 ppm by mass or less with respect to the total of the slurry. With the total content of iron and manganese in the slurry falling within this range, it is anticipated that the concentration of heavy metals within the slurry will also be low, thereby facilitating the suppression of crystallinity in the resulting magnesium glycinate.

The solid content concentration in the slurry is preferably 9.1% by mass or more and 41.7% by mass or less, more preferably 9.1% by mass or more and 35% by mass or less, and still more preferably 9.1% by mass or more and 30% by mass or less.

In the present disclosure, the solid content of the mixed solution means a heating residue after heating the mixed solution at 105°C for 4 hours, and the solid content concentration means a value obtained by dividing the total mass of the heating residue by the total mass of the mixed solution subjected to heating.

A dispersion medium in the slurry preferably comprises water. The content of the water is preferably 50% by mass or more and 100% by mass or less, more preferably 90% by mass or more and 100% by mass or less, and still more preferably 95% by mass or more and 100% by mass or less, with respect to the dispersion medium. The dispersion medium may comprise another solvent.

The dispersion medium may comprise alcohols such as methanol, ethanol, and isopropanol.

The slurry can be produced by mixing the magnesium raw material, glycine, and the dispersion medium.

The mixing time for glycine, magnesium raw material, and dispersing medium is preferably 10 minutes or longer and 10 hours or shorter, more preferably 30 minutes or longer and 6 hours or shorter, and most preferably 1 hour or longer and 3 hours or shorter. Mixing is considered to improve the uniformity of the slurry. Furthermore, shorter mixing time would more readily suppress the formation of substances that could act as crystal nuclei, thereby suppressing the crystallinity of the resulting magnesium glycinate.

The mixing temperature is preferably 5°C or more and 80°C or less, more preferably 10°C or more and 70°C or less, further preferably 15°C or more and 50°C or less, and even more preferably 20°C or more and 45°C or less. By maintaining the mixing temperature within the above range, dissolution of the raw materials in water is suppressed, the formation of substances that could act as crystal nuclei is suppressed, thereby more readily suppressing the crystallinity of the resulting magnesium glycinate.

The spray drying may be typically performed by a spray dryer. The spray drying conditions are not limited, but a slurry temperature immediately before spraying is preferably 15°C or more and 95°C or less, more preferably 20°C or more and 90°C or less, even more preferably 40°C or more and 90°C or less, and still more preferably 50°C or more and 90°C or less. By increasing the slurry temperature immediately prior to spraying, the formation of magnesium glycinate crystal nuclei is considered to be suppressed, thereby facilitating the suppression of the crystallinity of the resulting magnesium glycinate. In a preferred embodiment, the slurry temperature immediately before spraying is preferably 15°C or more and 95°C or less, more preferably 20°C or more and 80°C or less, further preferably 20°C or more and 60°C or less, and even more preferably 20°C or more and 45°C or less. Lowering the temperature immediately before spraying contributes to good reproducibility.

The obtained magnesium glycinate may be subjected to a drying treatment and/or a pulverization treatment.

An inlet drying temperature at the time of the drying treatment is not limited, and an outlet temperature may be an appropriate numerical value. The outlet temperature is preferably 50°C or more and 120°C or less, more preferably 60°C or more and 110°C or less, and still more preferably 80°C or more and 110°C or less. A drying time depends on the amount of liquid to be sprayed, and is not particularly limited as long as a product does not significantly adhere to the inside of a machine and the recovery of a powder is not difficult.

The drying may be performed using a spray dryer, a hot air dryer, a microwave dryer, or a rotary dryer or the like.

The pulverization can be performed so that the average particle diameter of the magnesium glycinate is preferably 1 µm or more and 350 µm or less, more preferably 10 µm or more and 300 µm or less, still more preferably 20 µm or more and 250 µm or less, and yet still more preferably 30 µm or more and 100 µm or less.

The pulverization may be performed using a fine pulverizer such as a roller mill, a jet mill, a high-speed rotary pulverizer, or a container-driven mill.

The magnesium glycinate of the present disclosure may be produced by the above production method, but is not limited to those produced by the above production method.

### (Third Embodiment: Magnesium Glycinate Tablet)

A magnesium glycinate tablet is also comprised within the technical scope of the present disclosure. In an aspect, the magnesium glycinate tablet of the present disclosure comprises the magnesium glycinate of the present disclosure. In another aspect, the magnesium glycinate tablet of the present disclosure exhibits a tensile strength of 100 N/cm² or more and 500 N/cm² or less when tableted at a tableting pressure of 5 kN per tablet.

The magnesium glycinate of the present disclosure exhibits good compression moldability, and it is expected that a tablet having a high magnesium glycinate concentration can be obtained. The magnesium glycinate tablet of the present disclosure is expected to have a high tensile strength and to be able to stably maintain the tablet.

The content of magnesium glycinate as a compound in the magnesium glycinate tablet is preferably 20% by mass or more and 95% by mass or less, more preferably 50% by mass or more and 95% by mass or less, and still more preferably 66.7% by mass or more and 90% by mass or less.

The content of the magnesium glycinate of the present disclosure in the magnesium glycinate tablet is preferably 0.1% by mass or more and 90% by mass or less, more preferably 10% by mass or more and 80% by mass or less, and still more preferably 20% by mass or more and 70% by mass or less.

The magnesium glycinate tablet may comprise, in addition to the magnesium glycinate, disintegrants such as low substituted hydroxypropyl cellulose, agar, croscarmellose sodium, partially pregelatinized starch, potato starch, corn starch, carmellose calcium, crospovidone, and sodium carboxystarch; binders such as crystalline cellulose, carboxypropyl cellulose, carboxypropyl methyl cellulose, and pullulan; lubricants such as a sucrose fatty acid ester, a glycerin fatty acid ester, sodium stearyl fumarate, talc, stearic acid, and salts thereof (Mg, Ca salts); fluidity improvers such as fine silicon dioxide and calcined shell calcium; sweetening agents such as aspartame, acesulfame potassium, and sucralose; and internal additives or external additives such as excipients, i.e., calcium hydrogen phosphate, a sugar alcohol, a monosaccharide, a disaccharide, an oligosaccharide, cellulose, a cellulose derivative, starch, a starch derivative, and a starch decomposition product. The magnesium glycinate tablet may further comprise other additives such as amino acids or salts thereof, yeast extracts, vitamins, minerals, functional components, various polyphenols, fixing agents, colorants, pH adjusting agents, buffers, and antioxidants.

The tensile strength of the magnesium glycinate tablet when tableted at a tableting pressure of 5 kN per tablet is preferably 100 N/cm² or more and 500 N/cm² or less, more preferably 100 N/cm² or more and 400 N/cm² or less, and still more preferably 100 N/cm² or more and 350 N/cm² or less. It is considered that the tensile strength of the magnesium glycinate tablet within such a range facilitates stably maintaining the tablet.

The shape of the magnesium glycinate tablet is not particularly limited, and may be, for example, a standard R, a two-stage R, a sugar-coated R, a corner R, a sharp-cornered plane, a rounded-corner plane, a triangle, an oblong shape, or a hexagon.

The size of the magnesium glycinate tablet is not particularly limited. In one aspect, the diameter of the magnesium glycinate tablet is preferably 5 mm or more and 14 mm or less, more preferably 5 mm or more and 10 mm or less, and still more preferably 5 mm or more and 9 mm or less. The thickness of the magnesium glycinate tablet is preferably 2 mm or more and 7 mm or less, 3 mm or more and 6.5 mm or less, or 3.5 mm or more and 6.5 mm or less. The weight per the magnesium glycinate tablet is preferably 50 mg or more and 1,000 mg or less, more preferably 70 mg or more and 800 mg or less, and still more preferably 90 mg or more and 600 mg or less.

The magnesium glycinate tablet can be produced by a production method comprising the steps of obtaining a mixed powder by mixing magnesium glycinate, and an internal additive, an external additive, and other additives to be used as necessary; and obtaining the magnesium glycinate tablet by tableting the obtained mixed powder. When the mixed powder is tableted, the mixed powder may be dispersed in a dispersion medium such as water and sprayed, or the mixed powder may be granular. A coating for the purpose of coloring the appearance to increase the commercial value or imparting a taste or scent may be added to the subsequent step.

The average particle diameter of the mixed powder may be preferably 100 µm or more and 850 µm or less, more preferably 150 µm or more and 500 µm or less, and still more preferably 150 µm or more and 300 µm or less.

The tableting method is not limited, but typically, tableting can be performed by using a tableting machine. For example, the tableting pressure per tablet is not limited, but is preferably 2 kN or more and 20 kN or less, more preferably 3 kN or more and 18 kN or less, and still more preferably 4 kN or more and 16 kN or less. As the pressure is higher, the hardness is higher, but the load on a pestle and a machine itself is higher, and the cost of repair is generated. Therefore, it is preferable to use the pressure as small as possible.

### (Fourth Embodiment: Pharmaceutical Product, or Food or Beverage Product)

The magnesium glycinate of the present disclosure may be suitably used for various applications. Examples of such applications include a food or beverage product and a pharmaceutical product, and the present disclosure also comprises a food or beverage product and a pharmaceutical product comprising the magnesium glycinate. The food or beverage product and the pharmaceutical product may be produced using the magnesium glycinate of the present disclosure.

The magnesium glycinate is considered to have a function of replenishing magnesium in the body by being absorbed in the body. By taking the magnesium, an effect of increasing a melatonin level in the brain, a relaxation effect, a good sleep effect, and an effect of stabilizing a blood glucose level, and the like are further expected. The magnesium glycinate is highly absorbed into the body, and has a suppressed laxative effect as a side effect, and thus can be preferably used as the food or beverage product and the pharmaceutical product.

In the present disclosure, the "food or beverage product" refers to a general food or beverage product, and comprises general foods such as health food; foods with functional claims; health functional foods such as foods for specified health uses and nutritive functional foods; and supplements. The "food or beverage product" is not limited to those administered to humans, and comprises livestock feeds and pet foods administered to animals, and the like.

The form of the food or beverage product is not particularly limited, and may be any of a solid, a semi-solid, and a liquid. Specific forms include tablets, pills, capsules, solutions, pastes, syrups, powders, granules, gummy and gel.

Also, the "pharmaceutical product" may be administered to a subject in any form and may be administered orally or parenterally. Preferably, the pharmaceutical product may be administered orally.

Such a pharmaceutical product may be used for the treatment of diseases such as magnesium deficiency, insomnia and depression, and various symptoms associated therewith.

The dosage form of the pharmaceutical product is not particularly limited, and examples thereof include oral solid preparations such as a tablet, a granule, a powder, and a capsule; oral liquid preparations such as an oral liquid preparation and a syrup; and parenteral liquid preparations such as an injection.

When the magnesium glycinate of the present disclosure is used for the food or beverage product, the food or beverage product may further comprise additives such as an excipient, a coating agent, a binder, an extender, a disintegrant, a surfactant, a lubricant, a diluent, a dispersant, a buffer, an osmotic pressure regulator, a pH regulator, an emulsifier, a preservative, a stabilizer, an antioxidant, a colorant, an ultraviolet absorber, a humectant, a thickener, an activity enhancer, an anti-inflammatory agent, a bactericide, a flavoring agent, and an odor improving agent.

The food or beverage product or the pharmaceutical product comprising the magnesium glycinate of the present disclosure may be used by administering it to a subject.

The magnesium glycinate of the present disclosure exhibits good compression deformability. The tensile strength of the tablet obtained from the magnesium glycinate of the present disclosure is good. Therefore, it is expected that the magnesium glycinate is tableted to obtain a tablet, and the tablet can be stably maintained. Therefore, the magnesium glycinate of the present disclosure is preferably used as the food or beverage product and the pharmaceutical product. The magnesium glycinate of the present disclosure is also preferably used in a method for producing the food or beverage product or the pharmaceutical product.

### EXAMPLES

The present disclosure will be described more specifically with reference to the following examples, but the present disclosure is not limited thereto.

### (test example 1-1)

18.0 g of glycine (special grade, FUJIFILM Wako Pure Chemical Corporation, γ type crystal) and 7.0 g of magnesium hydroxide (iron content 86.7 ppm by mass, manganese content 14.8 ppm by mass, the same applies hereinafter unless otherwise mentioned) were weighed, and added simultaneously to 50 mL of ion-exchanged water, followed by stirring in a water bath at room temperature (22 to 26°C) for 18 hours (As One, VOLTEGA, Power Stirrer VPS-160BD). The obtained slurry was spray-dried with a spray dryer (Yamato Scientific Co., Ltd., PARVIS MINI SPRAY GB210A) while being stirred at room temperature (22 to 26°C) to collect 3.2 g of magnesium glycinate powder. At the time of spray drying, the conditions were as follows: inlet temperature: 180°C, outlet temperature: 78 to 82°C, and air flow rate: 0.4 to 0.5 m³/min.

### (test example 1-2)

3.5 g of magnesium glycinate powder was obtained by powderizing in the same manner as in test example 1-1 except that the glycine was charged into ion-exchanged water, followed by stirring for 1 hour, and the magnesium hydroxide was then added thereto.

### (test example 1-3)

5.5 g of magnesium glycinate powder was obtained by powderizing in the same manner as in test example 1-1 except that 0.5 g of magnesium oxide (iron content 176 ppm by mass, manganese content 27.7 ppm by mass, the same applies hereinafter unless otherwise mentioned) and 6.5 g of the magnesium hydroxide were used in place of the magnesium hydroxide alone.

### (test example 1-4)

8.0 g of magnesium glycinate powder was obtained by powderizing in the same manner as in test example 1-2 except that 1 g of the magnesium oxide and 6 g of the magnesium hydroxide were used instead of the magnesium hydroxide alone.

### (test example 1-5)

5.6 g of magnesium glycinate powder was obtained by powderizing in the same manner as in test example 1-2 except that 1.6 g of the magnesium oxide and 5.4 g of the magnesium hydroxide were used instead of the magnesium hydroxide alone.

### (test example 1-6)

6.4 g of magnesium glycinate powder was obtained by powderizing in the same manner as in test example 1-2 except that 2.2 g of the magnesium oxide and 4.8 g of the magnesium hydroxide were used instead of the magnesium hydroxide alone.

### (test example 1-7)

6.0 g of magnesium glycinate powder was obtained by powderizing in the same manner as in test example 1-2 except that 2.9 g of the magnesium oxide and 4.1 g of the magnesium hydroxide were used instead of the magnesium hydroxide alone.

### (test example 1-8)

5.9 g of magnesium glycinate powder was obtained by powderizing in the same manner as in test example 1-2 except that 3.6 g of the magnesium oxide and 3.4 g of the magnesium hydroxide were used instead of the magnesium hydroxide alone.

### (test example 1-9)

7.1 g of magnesium glycinate powder was obtained by powderizing in the same manner as in test example 1-2 except that 4.3 g of the magnesium oxide and 2.7 g of the magnesium hydroxide were used instead of the magnesium hydroxide alone.

### (test example 1-10)

2.6 g of magnesium glycinate powder was obtained by powderizing in the same manner as in test example 1-2 except that 5.1 g of the magnesium oxide and 1.9 g of the magnesium hydroxide were used instead of the magnesium hydroxide alone.

### (test example 1-11)

6.1 g of magnesium glycinate powder was obtained in the same manner as in test example 1-2 except that the liquid amount of ion-exchanged water was changed to 75 mL.

### (test example 1-12)

9.2 g of magnesium glycinate powder was obtained in the same manner as in test example 1-2 except that the liquid amount of ion-exchanged water was changed to 125 mL.

### (test example 1-13)

11.0 g of magnesium glycinate powder was obtained in the same manner as in test example 1-2 except that the liquid amount of ion-exchanged water was changed to 250 mL.

### (test example 1-14)

5.8 g of magnesium glycinate powder was obtained in the same manner as in test example 1-6 except that the liquid amount of ion-exchanged water was changed to 35 mL.

### (test example 1-15)

A slurry solution was prepared in the same manner as in test example 1-6, then heated at 50°C for 1 hour, and powderized in the same manner as in test example 1-6 while the temperature was maintained, to obtain 4.2 g of magnesium glycinate powder.

### (test example 1-16)

A slurry solution was prepared in the same manner as in test example 1-6, then heated at 80°C for 1 hour, and powderized in the same manner as in test example 1-6 while the temperature was maintained, to obtain 5.4 g of magnesium glycinate powder.

### (test example 1-17)

5.6 g of magnesium glycinate powder was obtained by powderizing in the same manner as in test example 1-16 except that the amount of ion-exchanged water was 75 mL.

### (test example 1-18)

6.1 g of magnesium glycinate powder was obtained by powderizing in the same manner as in test example 1-17 except that an outlet temperature during spray drying was 98 to 102°C.

### (test example 1-19)

6.5 g of magnesium glycinate powder was obtained by powderizing in the same manner as in test example 1-2 except that 19.71 g of the glycine was charged into ion-exchanged water, followed by stirring for 30 minutes, and 5.29 g of magnesium oxide (iron content 91 ppm by mass, manganese content 31 ppm by mass) was then added thereto.

### (test example 1-20)

7.2 g of magnesium glycinate powder was obtained by powderizing in the same manner as in test example 1-19 except that magnesium oxide (iron content 177 ppm by mass, manganese content 62 ppm by mass) was used.

### (test example 1-21)

8.2 g of magnesium glycinate powder was obtained by powderizing in the same manner as in test example 1-19 except that magnesium oxide (iron content 176 ppm by mass, manganese content 27.7 ppm by mass) was used and the stirring time after charging the magnesium oxide was set at 6 hours.

### (test example 1-22)

50 mL of ion-exchanged water was heated to 80 °C, and 19.71 g of α-type crystal (Glycine G, Kobe Chemical) was charged, followed by stirring for 30 minutes, then magnesium oxide (iron content 5 ppm by mass, manganese content 0 ppm by mass) was added thereto, and stirring was kept for 2 hours. The reaction slurry was cooled down to 40 °C to obtain 6.4 g of magnesium glycinate by powderizing in the same manner as in test example 1-2.

### (test example 1-23)

6.0 g of magnesium glycinate powder was obtained by powderizing in the same manner as in test example 1-22 except that γ-type crystal was used as the glycine, and magnesium oxide (iron content 176 ppm by mass, manganese content 27.7 ppm by mass) was used as the magnesium oxide.

### (comparative example 1)

2.1 g of magnesium glycinate was obtained by powderizing in the same manner as in test example 1-1 except that 19.7 g of the glycine was used, and magnesium oxide (iron content 176 ppm by mass, manganese content 27.7 ppm by mass) was used instead of the magnesium hydroxide.

The powders obtained in the above Test Examples were subjected to the following measurements.

### (Powder X-ray Diffraction Measurement)

The measurement was performed using an X-ray diffractometer EMPYREAN (Malvern Panalytical). A measurement sample was filled into a circular sample holder manufactured by Malvern Panalytical without gaps using a sample preparation kit manufactured by Malvern Panalytical. The measurement conditions are as follows.
X-ray source: CuKα (λ=1.5418 Å)
Acceleration voltage: 45 kV
Step: 2θ=0.026°
Scan speed: 0.656514°/s
Divergence slit: 0.5000°
Scattering slit: 8.0 mm
Soller slit: 0.04 rad

The obtained XRD diffraction pattern was analyzed by peak detection and peak fitting using HighScore Plus (Malvern Panalytical). The phase and FWHM of a peak top were calculated from the obtained peak, and a crystallite size was calculated using the following equation. The FWHM here is not applied to the calculation of a crystallinity.
D = Kλ/Bcosθ
λ = 1.5418 Å
K = 0.9
B: FWHM
θ: phase of peak top

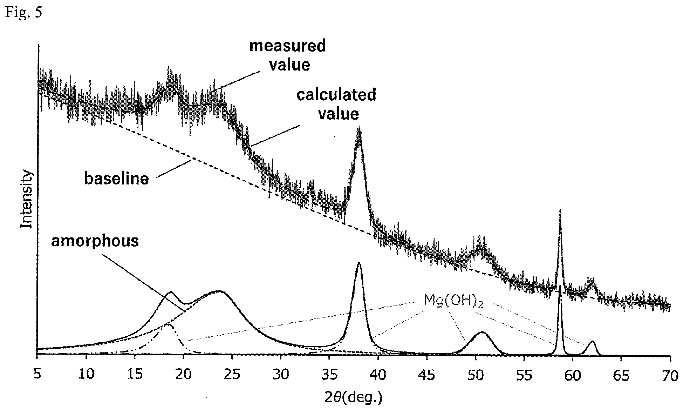

Furthermore, in order to calculate the crystallinity, JADE (MDI) of XRD diffraction pattern analysis software was used. As the XRD diffraction pattern, data obtained by measurement with the above-described X-ray diffractometer was used. The diffraction pattern was loaded into the JADE, and a baseline was set as a quartic equation. An amorphous peak was defined so that a peak was set in a range of a diffraction angle of 20 to 30°. The peak fitting was performed to obtain the peak position, area, and full width at half maximum of each peak. With reference to the obtained peak area, a crystallinity CI for each XRD diffraction pattern was calculated from the following equation. A representative example illustrating a calculation principle is illustrated in Fig. 5.$CI = S 2 / \left(S1 + S2\right)$
S1: the area of an amorphous peak having a maximum value in a range of 20° ≤ 2θ ≤ 30° and a full width at half maximum of 2° or more
S2: an area obtained by subtracting the sum of areas of peaks attributed to magnesium hydroxide in a range of 5° ≤ 20 ≤ 70° from the sum of areas of crystal peaks having a maximum value in a range of 5° ≤ 20 ≤ 70° and a full width at half maximum of less than 1°

### (Thermogravimetric Analysis)

The measurement was performed using a thermogravimetric analyzer STA2500 Regulus (NETZSCH-Geratebau GmbH). The measurement conditions are as follows.
Sample amount: 10 to 11 mg
Sample container: aluminum pan
Heating rate: heated from 30°C to 600°C at 10°C/min
Atmospheric gas: nitrogen gas (50 mL/min)
Control substance: alumina (10 to 11 mg)

### (ICP emission spectroscopy)

Iron and manganese contents were analysed by ICP emission spectroscopy. Specifically, the test samples were first heated to 500°C or above in an electric furnace to incinerate them. Iron and manganese were then extracted separately from the ash in hydrochloric acid. The extracts were filtered to remove insoluble solids, made up to volume in volumetric flasks, and quantitatively analysed by ICP emission spectroscopy.

### (Measurement of Tensile Strength)

The tablet produced in each Test Example had a cylindrical shape, and a thickness [mm] in a direction of sandwiching the circular surface was measured using a commercially available general-purpose digital thickness gauge. The hardness [N] of the tablet was measured using a tablet hardness tester (MODEL 6D TABLET TESTER manufactured by DR. SCHLEUNIGER) so that a force acts in a horizontal direction on a circular surface, and the measurement was repeated three times for each. The obtained value was converted into a tensile strength using the following equation. $Tensile strength \left[N/{cm}^{2}\right] = 2 \times hardness \left[N\right] / \left(π\times 0.1 cm\times thickness of tablet \left[cm\right]\right)$

For Test Examples 1-1 to 22 and Comparative Example 1, the molar ratio of magnesium oxide to magnesium hydroxide (MgO:Mg(OH)₂) in the magnesium raw material, heavy metal content (Fe content, Mn content), slurry concentration and slurry temperature immediately prior to spraying, glycine retention rate in the resulting magnesium glycinate, and the rate of weight loss at 400°C relative to the weight at 50°C (TG reduction rate) in thermogravimetric analysis, as well as the degree of crystallinity, are shown in the table below.

**[Table 1]**

| test example No. | Molar ratio | Heavy metal content [µg] | | | Heavy metal concentration [ppm by mass] | | | Slurry concentration [w/w %] | Slurry temperature immediately before spraying | Glycine residual ratio [% by mass] | TG weight reduction rate [% by mass] | Crystallinity |
|---|---|---|---|---|---|---|---|---|---|---|---|---|
| | | Fe | Mn | Fe+Mn | Fe | Mn | Fe+Mn | | | | | |
| 1-1 | 0:10 | 606.9 | 103.6 | 710.5 | 86.7 | 14.8 | 101.5 | 33.3% | 22-26°C | 6.30 | 33.7 | 0.000 |
| 1-2 | 0:10 | 606.9 | 103.6 | 710.5 | 86.7 | 14.8 | 101.5 | 33.3% | 22-26°C | 6.93 | 34.9 | 0.000 |
| 1-3 | 1:9 | 651.6 | 110.1 | 761.6 | 93.1 | 15.7 | 108.8 | 33.3% | 22-26°C | 6.87 | 31.1 | 0.000 |
| 1-4 | 2:8 | 696.2 | 116.5 | 812.7 | 99.5 | 16.6 | 116.1 | 33.3% | 22-26°C | 7.48 | 31.6 | 0.325 |
| 1-5 | 3:7 | 749.8 | 124.2 | 874.0 | 107.1 | 17.7 | 124.8 | 33.3% | 22-26°C | 7.86 | 32.6 | 0.629 |
| 1-6 | 4:6 | 803.4 | 132.0 | 935.3 | 114.8 | 18.9 | 133.7 | 33.3% | 22-26°C | 6.64 | 30.4 | 0.000 |
| 1-7 | 5:5 | 865.9 | 141.0 | 1006.9 | 123.7 | 20.1 | 143.8 | 33.3% | 22-26°C | 7.51 | 31.5 | 0.515 |
| 1-8 | 6:4 | 928.4 | 150.0 | 1078.4 | 132.6 | 21.4 | 154.0 | 33.3% | 22-26°C | 7.34 | 32.2 | 0.705 |
| 1-9 | 7:3 | 990.9 | 159.1 | 1150.0 | 141.6 | 22.7 | 164.3 | 33.3% | 22-26°C | 4.23 | 31.0 | 0.835 |
| 1-10 | 8:2 | 1062.3 | 169.4 | 1231.7 | 151.8 | 24.2 | 176.0 | 33.3% | 22-26°C | 1.29 | 30.1 | 0.810 |
| 1-11 | 0:10 | 606.9 | 103.6 | 710.5 | 86.7 | 14.8 | 101.5 | 25.0% | 22-26°C | 6.70 | 32.6 | 0.000 |
| 1-12 | 0:10 | 606.9 | 103.6 | 710.5 | 86.7 | 14.8 | 101.5 | 16.7% | 22-26°C | 5.87 | 33.2 | 0.000 |
| 1-13 | 0:10 | 606.9 | 103.6 | 710.5 | 86.7 | 14.8 | 101.5 | 9.1% | 22-26°C | 6.11 | 33.3 | 0.000 |
| 1-14 | 4:6 | 803.4 | 132.0 | 935.3 | 114.8 | 18.9 | 133.7 | 41.7% | 22-26°C | 6.25 | 34.0 | 0.730 |
| 1-15 | 4:6 | 803.4 | 132.0 | 935.3 | 114.8 | 18.9 | 133.7 | 33.3% | 50°C | 6.18 | 35.7 | 0.000 |
| 1-16 | 4:6 | 803.4 | 132.0 | 935.3 | 114.8 | 18.9 | 133.7 | 33.3% | 80°C | 3.64 | 39.4 | 0.000 |
| 1-17 | 4:6 | 803.4 | 132.0 | 935.3 | 114.8 | 18.9 | 133.7 | 25.0% | 80°C | 3.07 | 29.9 | 0.000 |
| 1-18 | 4:6 | 803.4 | 132.0 | 935.3 | 114.8 | 18.9 | 133.7 | 25.0% | 80°C | 1.54 | 39.9 | 0.000 |
| 1-19 | 10:0 | 481.4 | 164.0 | 645.4 | 91.0 | 31.0 | 122.0 | 33.3% | 22-26°C | 6.64 | 31.0 | 0.000 |
| 1-20 | 10:0 | 936.3 | 328.0 | 1264.3 | 177.0 | 62.0 | 239.0 | 33.3% | 22-26°C | 3.07 | 38.7 | 0.000 |
| 1-21 | 10:0 | 931.0 | 146.5 | 1077.6 | 176.0 | 27.7 | 203.7 | 33.3% | 22-26°C | 2.21 | 31.7 | 0.890 |
| 1-22 | 10:0 | 26.5 | 0.0 | 26.5 | 5.0 | 0.0 | 5.0 | 33.3% | 40°C | 2.43 | 34.8 | 0.990 |
| 1-23 | 10:0 | 931.0 | 146.5 | 1077.6 | 176.0 | 27.7 | 203.7 | 33.3% | 40°C | 4.47 | 33.0 | 0.860 |
| comparative example 1 | 10:0 | 931.0 | 146.5 | 1077.6 | 176.0 | 27.7 | 203.7 | 33.3% | 22-26°C | 1.85 | 31.7 | 1.000 |

In test examples 1-1 to 1-10, no differences in the results were observed between the cases of adding glycine and magnesium hydroxide simultaneously versus adding them separately. As the proportion of magnesium oxide in the magnesium raw material was increased, a completely amorphous product was obtained (crystallinity: 0.000) at molar ratios (magnesium oxide: magnesium hydroxide) of 0:10, 1:9, and 4:6. At other ratios, a mixture of dihydrate crystals and amorphous material was obtained. Particularly in Test Examples 1-7 to 1-10, where the molar ratio (magnesium oxide: magnesium hydroxide) ranged from 5:5 to 8:2, and in Comparative Example 1 with a molar ratio of 10:0, there was a tendency for the proportion of crystalline material to increase.

In test examples 1-11 to 1-14, it was shown that the lower the slurry concentration, the more the glycine residual amount tends to decrease, and the higher the chelation efficiency. Meanwhile, it was shown that regarding the crystallization rate, the amorphous is changed to the mixture of the dihydrate crystal and the amorphous when the slurry concentration increases from 33.3% to 41.7%.

In test examples 1-6, 15-18, by increasing the slurry temperature immediately before spraying to 80°C, the glycine residual ratio was reduced while the amorphous state was maintained, and thus it was considered that the chelation reaction was promoted. This tendency was also considered to have been promoted by lowering the slurry concentration and changing the crystalline form of the glycine from a stable γ type to a metastable α type, and the amorphous existent state was maintained.

Furthermore, when the outlet temperature was increased to about 100°C under the spray conditions, heat was applied to the obtained powder, and the glycine residual ratio decreased while the crystallinity was maintained at 0. The finding that the chelation efficiency was improved while the amorphous state was maintained as described above has not been found in the previous report, and was first perceived by this experiment.

In test examples 1-19 and 20, it was confirmed that even when the amount of heavy metals contained in the magnesium raw material increased, the amorphous state was maintained when the stirring time after adding the magnesium raw material was set to 2 hours. Conversely, in test example 1-21, when the stirring time after adding the magnesium raw material was set to 6 hours, it was confirmed that crystallinity increased compared to test example 1-20, despite the magnesium raw material containing a lower quantity of heavy metals.

Comparing test examples 1-8 and 21, it is considered that the crystallinity of the magnesium glycinate obtained tends to increase with a higher content of magnesium oxide in the magnesium raw material. The reason for this is that magnesium oxide has higher reactivity with glycine. Additionally, the following factors may be considered. Namely, during the spray-drying process, it is thought that the molecules of glycine and the magnesium raw material are instantaneously fixed and become amorphous. Consequently, the extent of crystal growth may depend on the presence of crystal nuclei within the slurry subjected to spray drying. Considering these aspects, when magnesium oxide is used as the magnesium raw material, the time to reach saturation solubility is shorter, facilitating the formation of magnesium glycinate crystal nuclei. This may explain the enhanced crystallinity observed when magnesium oxide is employed. This point is consistent with the higher crystallinity of the magnesium glycinate obtained in test examples 1-14, where the slurry concentration was high, as it is thought that reaching the saturation solubility point facilitates the formation of crystal nuclei.

Comparing Test Examples 1-22 and 1-23, it was confirmed that changing the crystalline form of glycine from the stable γ-type to the metastable α-type enhances crystallinity, even under conditions favoring an amorphous state characterized by low content of the heavy metal and short reaction times. This effect is considered to arise from α-type crystalline glycine promoting chelation reactions and reducing unreacted material, thereby increasing crystallinity. Furthermore, as chelation was promoted, it is thought that the residual glycine content could also be reduced.

### (examples 2-1, 2, 3, comparative example 2)

Tablets comprising the following formulation of magnesium glycinate from test examples 1-17 (crystallinity: 0.000), test example 1-4 (crystallinity: 0.325), and test example 1-10 (crystallinity: 0.810) were used as examples 2-1, 2-2, and 2-3. Furthermore, the case using a commercially available crystalline dihydrate raw material containing no amorphous phase (Actylis, Magnesium Bisglycinate) was designated as comparative example 2 (crystallinity: 1.000). At this time, crystalline cellulose (Asahi Kasei Corporation's Theoras UF-F702), fine-particle silicon dioxide (Fuji Silicia's Silopage 720), and calcium stearate (Taihei Chemical Co., Ltd.) were used, and the formulation shown in the table below was prepared. The total weight was set to 4.5 g, the respective raw materials were mixed, and tableting was performed the following day.

For the commercially available crystalline raw material (Acetylis, Magnesium Bisglycinate) not containing amorphous components, the weight loss rate at 400°C relative to the weight at 50°C, as determined by thermogravimetric analysis, was 45.8% by mass.

The tableting test was conducted using a Type-M static compressor manufactured by Maekawa Test Equipment Co., Ltd. A 10 mm diameter, perfectly flat, circular punch and its corresponding die were fitted to the compressor. Each cavity was filled with 300 mg of material and compressed. Compression pressures of 5, 10, and 15 kN were applied, with each test repeated three times.

**[Table 2]**

| | example 2-1 | example 2-2 | example 2-3 | comparative example 2 |
|---|---|---|---|---|
| | Blending amount per tablet | | | |
| Magnesium glycinate | 200 Use of test example 1-17 | 200 Use of test example 1-4 | 200 Use of test example 1-10 | 200 Use of commercially available product |
| Crystalline cellulose | 94 | 94 | 94 | 94 |
| Fine silicon dioxide | 3 | 3 | 3 | 3 |
| Calcium stearate | 3 | 3 | 3 | 3 |
| Total weight | 300 | 300 | 300 | 300 |

| | | | | |
|---|---|---|---|---|
| [unit mg] | | | | |

For the purpose of evaluating the compression moldabilities of magnesium glycinates having different crystallinities, tablets were produced, and the results of evaluating the strengths as the tensile strength are shown in Table 3.

In all Test Examples, the tensile strength was improved as the tableting pressure increased, but the tensile strength decreased as the crystallinity increased. In general, it is considered that the tensile strength of about 100 N/cm² can withstand distribution. A tablet using magnesium glycinate having a crystallinity of 0 to 0.81 had a sufficient hardness in spite of a high content of 66.7%. Meanwhile, Comparative Example of a commercially available product was an amorphous-free dihydrate crystal. The strength of a tablet produced using the dihydrate crystal was decreased, and practically, Comparative Example could not be formed into a tablet.

**[Table 3]**

| Types of Examples | | example 2-1 | example 2-2 | example 2-3 | comparative example 2 |
|---|---|---|---|---|---|
| Crystallinity | | 0.000 | 0.325 | 0.810 | 1.000 |
| Tensile strength [N/cm²] | Tableting pressure 5 kN | 316.4 | 178.9 | 113.5 | 72.3 |
| | Tableting pressure 10 kN | 783.9 | 470.6 | 327.0 | 175.7 |
| | Tableting pressure 15 kN | 1038.8 | 695.7 | 488.7 | 279.8 |

### Industrial Applicability

The magnesium glycinate of the present disclosure has good compression deformability. The tensile strength of the tablet obtained from the magnesium glycinate of the present disclosure is good. Therefore, it is expected that the magnesium glycinate is tableted to obtain a tablet, and the tablet can be stably maintained. Therefore, the magnesium glycinate of the present disclosure is preferably used as the food or beverage product and the pharmaceutical product. The magnesium glycinate of the present disclosure is also preferably used in a method for producing the food or beverage product or the pharmaceutical product.

## Claims

1. Magnesium glycinate having a crystallinity represented by S2/(S1 + S2) of 0 or more and 0.9 or less,
wherein in an X-ray powder diffraction spectrum,
S1 is an area of an amorphous peak having a maximum value in a range of 20° ≤ 2θ ≤ 30° and a full width at half maximum of 2° or more, and
S2 is an area obtained by subtracting a sum of areas of peaks attributed to magnesium hydroxide in a range of 5° ≤ 2θ ≤ 70° from a sum of areas of crystal peaks having a maximum value in a range of 5° ≤ 2θ ≤ 70° and a full width at half maximum of less than 1°.

2. The magnesium glycinate according to claim 1, wherein a weight reduction rate at 400°C based on a weight at 50°C in thermogravimetric analysis is 40% by mass or less.

3. The magnesium glycinate according to claim 1, wherein the crystallinity is 0 or more and 0.81 or less.

4. A food or beverage product, or a pharmaceutical product comprising the magnesium glycinate according to any one of claims 1 to 3.

5. A method for producing a food or beverage product, or a pharmaceutical product, comprising using the magnesium glycinate according to any one of claims 1 to 3.

6. A magnesium glycinate tablet comprising the magnesium glycinate according to any one of claims 1 to 3.

7. A magnesium glycinate tablet having a tensile strength of 100 N/cm² or more and 500 N/cm² or less when tableted at a tableting pressure of 5 kN per tablet.

8. A method for producing magnesium glycinate comprising the step of obtaining the magnesium glycinate by reacting glycine with a magnesium raw material,
wherein the magnesium raw material comprises one or more selected from magnesium hydroxide and magnesium oxide.

9. A method for producing magnesium glycinate comprising the step of obtaining the magnesium glycinate by spray-drying a slurry comprising glycine and a magnesium raw material,
wherein the magnesium raw material comprises one or more selected from magnesium hydroxide and magnesium oxide,
wherein a solid content concentration of the slurry is 9.1% by mass or more and 41.7% by mass or less, and
wherein a slurry temperature immediately before spraying is 20°C or higher and 90°C or lower.

10. The method for producing magnesium glycinate according to claim 8 or 9, wherein the magnesium raw material comprises magnesium hydroxide.

11. The method for producing magnesium glycinate according to claim 8 or 9, wherein the total content of iron and manganese in the magnesium raw material is 0 ppm by mass or more and 270 ppm by mass or less with respect to the total of the magnesium raw material.

12. The method for producing magnesium glycinate according to claim 8 or 9, wherein the magnesium raw material comprises magnesium hydroxide and magnesium oxide, and a content of the magnesium hydroxide is 20% by mass or more in the magnesium raw material.
